# EUROPEAN PATENT SPECIFICATION

(11) **EP 4 192 449 B1**
(45) Date of publication and mention of the grant of the patent: **19.06.2024**
(21) Application number: 21752232.5
(22) Date of filing: 02.08.2021
(51) Int. Cl.: A61K 31/397, A61P 35/00, C07D 205/08

(54) **MODIFIED EZETIMIBE DRUG FOR CANCER TREATMENT**
MODIFIZIERTES EZETIMIB-ARZNEIMITTEL ZUR KREBSBEHANDLUNG
MÉDICAMENT D'ÉZÉTIMIBE MODIFIÉ POUR LE TRAITEMENT DU CANCER

(30) Priority: 04.08.2020 ZA 202004811
(43) Date of publication of application: 14.06.2023
(73) Proprietor: University of South Africa, 0001 Pretoria (ZA)
(72) Inventor: NTWASA, Monde, 2193 Johannesburg (ZA); TWALA, Charmy, Starnod, 0380 Thabazimbi (ZA)
(74) Representative: Potter Clarkson
(86) International application number: PCT/IB2021/057046
(87) International publication number: WO 2022/029598

(56) References cited:
- WO-A1-2021/094933
- Charmy Starnod Twala: "DRUGS TARGETING THE RETINOBLASTOMA BINDING PROTEIN 6 (RBBP6)" In: "DRUGS TARGETING THE RETINOBLASTOMA BINDING PROTEIN 6 (RBBP6)", 3 November 2017 (2017-11-03), Faculty of Science, University of the Witwatersrand, Johannesburg, XP055769840, page 47 - page 50 page 60 page 62 - page 63
- SIROUS HAJAR ET AL: "An integrated in silico screening strategy for identifying promising disruptors of p53-MDM2 interaction", COMPUTATIONAL BIOLOGY AND CHEMISTRY, ELSEVIER, AMSTERDAM, NL, vol. 83, 16 August 2019 (2019-08-16), XP085924938, ISSN: 1476-9271, DOI: 10.1016/J.COMPBIOLCHEM.2019.107105 [retrieved on 2019-08-16]
- AYDIN GULSAH ET AL: "Proposing novel MDM2 inhibitors: Combined physics-driven high-throughput virtual screening and in vitro studies", CHEMICAL BIOLOGY & DRUG DESIGN, vol. 96, no. 1, 1 July 2020 (2020-07-01), pages 684-700, XP055850363, ISSN: 1747-0277, DOI: 10.1111/cbdd.13694 Retrieved from the Internet: URL:https://onlinelibrary.wiley.com/doi/fu ll-xml/10.1111/cbdd.13694>
- LIU YINGLE ET AL: "4-CF3-ezetimibe analogs: design, synthesis, and biological evaluation of cholesterol absorption inhibitions", TETRAHEDRON LETTERS, vol. 54, no. 40, 13 August 2013 (2013-08-13), pages 5541-5543, XP028704921, ISSN: 0040-4039, DOI: 10.1016/J.TETLET.2013.08.027
- SWALLOW ET AL: "Fluorine in Medicinal Chemistry", PROGRESS IN MEDICINAL CHEMISTRY, ELSEVIER, AMSTERDAM, NL, vol. 54, 1 January 2015 (2015-01-01), pages 65-133, XP009529600, ISSN: 0079-6468, DOI: 10.1016/BS.PMCH.2014.11.001 [retrieved on 2015-01-17]

## Description

### FIELD OF THE INVENTION

THIS INVENTION relates to an anti-cancer drug that is a modification of the drug ezetimibe, the modified drug having improved drug-like properties and being less vulnerable to metabolic enzymes than ezetimibe.

### BACKGROUND TO THE INVENTION

The tumour suppressor protein p53 is involved in a number of important biological process that are crucial for carcinogenesis, including the cell cycle, apoptosis, DNA repair, angiogenesis, glucose metabolism and innate immunity. It is a transcription factor that acts as a regulator of the oncogene Mouse Double Minute (Mdm2) in an auto regulatory feedback loop. p53 activates expression of the Mdm2 gene and Mdm2 regulates p53 by controlling its transport out of the nucleus thereby making it unavailable to its gene targets. This inhibits its transcription function or promotes its degradation by proteasomes using ubiquitin-ligase activity.

Mdm2 has a hydrophobic binding pocket to which p53 binds by means of a peptide in its transactivation domain. This pocket is a key target for drugs that inhibit the p53-Mdm2 interaction. Small molecular drug design has attempted to formulate small drug-like molecules that can competitively target the Mdm2 p53-binding domain, disrupting the formation of Mdm2-p53 complexes and thus increasing the levels of reactive p53 in cancer cells to promote a p53-dependent cell death. These drug design studies have resulted in the synthesis of nutlins (nutlin-2, nutlin-3a and MI-219), of which nutlin-3a is the most potent with a very low toxicity profile (IC₅₀ 0.09µM).

The applicant has previously shown that ezetimibe can bind more strongly than nutlins to the Mdm2 hydrophobic pocket. Furthermore, the applicant has shown that ezetimibe is toxic to cancer cell lines, especially those that overexpress Mdm2. While ezetimibe does not structurally resemble nutlins, based on molecular docking simulations ezetimibe accurately mimics p53 binding to the Mdm2 hydrophobic cleft. Ezetimibe, however, has structural vulnerabilities that hinder its use as an anti-cancer agent, such as its conversion by metabolic enzymes in the intestines. This results in negligible bioavailability of ezetimibe in the intestines and as such it is unsuitable for use in the treatment of colonic cancers.

In order to address this, the applicant was prompted to modify ezetimibe in order to decrease its degradation by metabolic enzymes in the intestine. This was achieved by replacing a hydroxyl group, which is a site for glucuronidation, in the ezetimibe lead molecule with a fluorine. This new drug, MC011019, has improved drug-like properties. The modification prevents metabolic transformation of the drug in the intestine and increases its bioavailability in the intestine, which makes it suitable for treating colon and colorectal cancers, and potentially other cancers that overexpress Mdm2.

### DISCLOSURE OF THE INVENTION

According to a first aspect of the invention there is provided a compound having the structure of Formula (I): 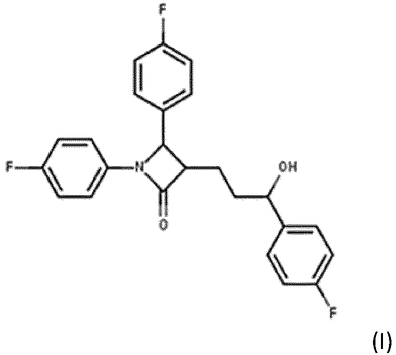 or a pharmaceutically acceptable salt thereof.

The compound or a pharmaceutically acceptable salt thereof may bind tightly to Mdm2, and in particular to the hydrophobic binding pocket of Mdm2 to which p53 binds. The compound or a pharmaceutically acceptable salt thereof may therefore prevent the binding of Mdm2 to p53, thereby increasing the levels of p53 in a cell.

The compound or a pharmaceutically acceptable salt thereof may have good bioavailability in the intestine as it may resist degradation, and in particular glucuronidation.

According to a second aspect of the invention there is provided a compound of Formula (I) or a pharmaceutically acceptable salt thereof for use in a method of treating a cancer.

The cancer may be a cancer in which there are elevated levels of Mdm2 or where Mdm2 is overexpressed and may include colon cancer, colorectal cancer, sarcoma, glioma, lymphoma, breast cancer, lung cancer, liver cancer, esophagogastric cancer and gynaecological cancers.

The compound or a pharmaceutically acceptable salt thereof may bind to the hydrophobic binding pocket of Mdm2 and increase the levels of active p53 in a cell. This may increase p53-mediated cell death of cancer cells.

Any references to methods of treatment by therapy in this description are to be interpreted as references to salts and pharmaceutical formulations of the present invention for use in those methods.

According to a third aspect of the invention there is provided use of a compound of Formula (I) or a pharmaceutically acceptable salt thereof in the manufacture of a medicament for treatment of a cancer, which may be a cancer in which Mdm2 levels are high or it is overexpressed.

The cancer may include colon cancer, colorectal cancer, sarcoma, glioma, lymphoma, breast cancer, lung cancer, liver cancer, esophagogastric cancer and gynaecological cancer.

The compound or a pharmaceutically acceptable salt thereof may bind to the hydrophobic binding pocket of Mdm2 to prevent the inhibition of p53 by Mdm2, thereby increasing p53-mediated cell death of cancer cells.

According to a fourth aspect of the invention there is provided a method of treating a cancer by administering the compound of Formula (I) or a pharmaceutically acceptable salt thereof to a patient in need thereof, wherein the cancer may be characterised by high levels or overexpression of Mdm2.

The compound or a pharmaceutically acceptable salt thereof may bind to the hydrophobic binding pocket of Mdm2 and increase the levels of p53 in the cell. The compound or a pharmaceutically acceptable salt thereof may promote p53-mediated cell death of cancer cells.

The cancer may be selected from colon cancer, colorectal cancer, sarcoma, glioma, lymphoma, breast cancer, lung cancer, liver cancer, esophagogastric cancer and gynaecological cancer.

### EXAMPLE

The invention will now be described in more detail with reference to the Example hereunder, and the accompanying drawings.

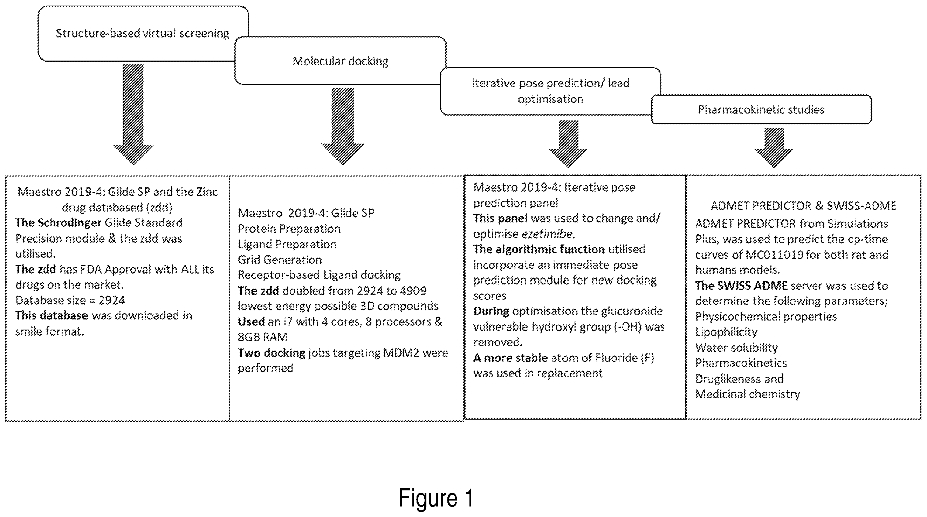

In the drawings
FIGURE 1 shows, for the Example, a summary of the adopted structure guided computer aided drug design methodology;
FIGURE 2 shows, for the Example, (A) ezetimibe and Mdm2 interaction and (B) a residue property surface along with a network of good steric contacts of the ezetimibe-Mdm2 complex;
FIGURE 3 shows, for the Example, the structural composition and interaction analysis of Mdm2 with ezetimibe with (A) being a ball and stick model of ezetimibe (ZINC03810860) and (B) being a 2D ligand interaction diagram with a collection of nearby residues and a single hydrogen bond co-ordination to VAL93;

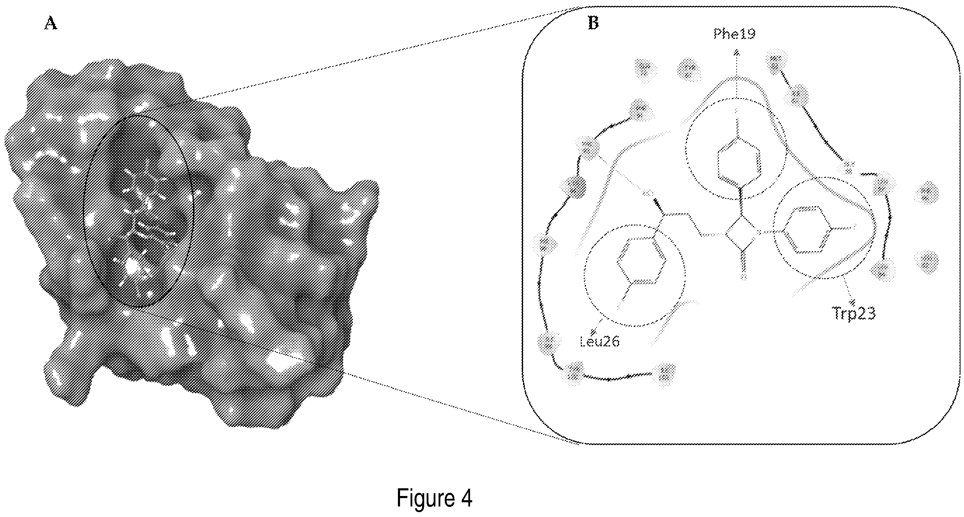

FIGURE 4 shows, the simulated surface presentation and interaction analysis of the Mdm2-MC011019 complex, with (A) being the Mdm2-MC011019 complex, where Mdm2 is shown in a grey surface area with a deep hydrophobic pocket and MC011019 is represented in ball and stick models; (B) being a 2D ligand interaction diagram with a collection of nearby residues at 3Å axis and a single hydrogen bond co-ordination to VAL93; and wherein the para fluoro-phenyl groups of MC011019 that are predicted to competitively inhibit the three critical residues of Phe19, Trp23 and Leu26 of the p53 transactivation domain are circled and labelled respectively;
FIGURE 5 shows pharmacokinetic (PK) properties of MC011019 (A) compared with ezetimibe (B);
FIGURE 6 shows, for the Example, a target prediction analysis with (A) being a summary of ezetimibe protein targets and (B) being a summary of possible MC011019 protein targets;
FIGURE 7 shows, for the Example, the pharmacokinetic profile in terms of cp-time curve analysis of MC011019 illustrating a prediction of the possible plasma concentration curve (cp-time curve) of MC011019 administered at different doses, i.e. 0.01mg (C), 0.1mg (B) and 1.0mg (A) using the rat as a model, the percentage of fraction absorbed (%Fa) and bioavailable (%Fb) as well as the C-max with its corresponding T-max;
FIGURE 8 shows, for the Example, the pharmacokinetic profile in terms of cp-time curve analysis of MC011019, illustrating a prediction of the possible plasma concentration curve (cp-time curve) of MC011019 administered at different doses, i.e. 1mg (C), 10mg (B) and 100mg (A) using a human model, the percentage of fraction absorbed (%Fa) and bioavailable (%Fb) as well as the C-max with its corresponding T-max; and
FIGURE 9 shows, for the Example, (A) the probability of MC011019 to interact off-target with various cellular proteins, and (B) the probability of ezetimibe to interact off-target with various cellular proteins.

### MATERIALS AND METHODS

### Protein and drug structures

The Mdm2 protein structure was downloaded from the Protein Database (PDB) in a pdb format and analysed using PyMol on which the p53 peptide was removed prior to docking studies. The drug ligands structures were retrieved from the Zinc Drug Database (Zdd) in a 2D configuration. The PubChem database was utilized to obtain the nutlin-3a drug structure in sdf format. The structure of ezetimibe was obtained from the DrugBank database in a SMILE format.

### Screening of chemical compounds and molecular docking

The Mdm2 p53-binding domain (Mdm2 p53BD) was used as a template on the Schrödinger's Maestro 2019-4: Glide SP (Standard Precision) application to screen the Zdd for chemical compounds that can target the Mdm2 p53BD. The Zdd constitutes commercially FDA approved drugs, available worldwide as pure compounds. In this study, the entire Zdd database of 2924 structures was screened. The use of Glide enabled both virtual screening and molecular docking studies to be done simultaneously. This application took the critical residues within the Mdm2 p53BD and the Zdd as inputs and generated a collection of chemical compounds docked into the specified pocket with different docking scores. The Schrödinger's Receptor-based ligand docking protocol employs a multi-step procedure, which involves the preparation and manipulation of the Mdm2 p53BD as well as ligands prior to screening and docking studies. These steps were sequentially performed as follows: Protein domain preparation, Ligand Preparation, Grid generation and Receptor-based ligand docking.

### • Protein preparation

The PDB structures are not suitable for immediate use in molecular modelling calculations, as they usually consist of only heavy atoms. They may also include core crystallized ligand, water molecules, metal ions and co-factors. Additionally, some structures are multi-meric and need to be reduced to a single unit, and because of the limited resolution of X-ray experiments it can be difficult to distinguish between the carbonyl oxygen and the secondary amine nitrogen of the amides in crystal structures thus the placement of the groups must be checked. PDB structures may also be missing atoms or connectivity information which must be assigned along with bond-orders and formal charges. Therefore, in this study the Schrödinger-Maestro v10.7 protein preparation wizard was used. This took Mdm2 p53BD from their raw state (having missing atoms or incorrect bond-order assignments, incorrect charge states and orientation of various groups) and brought them to a suitable state of being utilised by Glide. The wizard contains a Graphical User Interphase (GUI) with a systematic functional procedure.

### • Ligand preparation

The Zdd was downloaded (http://zinc12.docking.org/browse/subsets/special) in SMILE and SDF formats which contained 2D structures of chemical compounds. This configuration state is not suitable when performing molecular docking calculations, or to simulate using computational docking algorithms. Proteins exist in 3-dimensional space, thus drugs that would successfully target them should also exhibit such configuration. The Schrödinger-Maestro v10.7 ligand preparation wizard was used to convert 2924 2D structures into lowest energy possible 4909 3D structures in maestro format. This program allows for an expansion of each input structure by generating variations on the ionisation state, tautomers, stereochemistry, and ring conformations. The possible ionisation states of the ligands were generated at a target pH range of 7.0 +/- 2 using Epik which is a built-in application within Glide that predicts both the ionisation states and their associated penalties. Epik also predicts different tautomeric forms and calculates energy penalties for every ligand state it predicts. In Glide, the Epik state penalty is also used to differentiate active from inactive compounds during docking, in fact the use of Epik is known to improve virtual screening enrichment.

### • Grid generation

The outer scoring grids were generated with different dimensions ranging from 20×20×2Ǻ to 50×50×50Ǻ in the x, y, z - axis respectively. Generally, it is important to make the outer grid consistent with the shape of the protein's active site, thus this was done to only cover-up the active site volume of the Mdm2 p53-binding hydrophobic cleft. Literature-stipulated residues of the Mdm2 p53BD were used as a premise to accurately map-out critical residues facilitating the binding co-ordination of the p53 transactivation domain. A ligand centre box (inner grid) was generated to define the acceptable ligand centre positions during the side point search, providing a true measure of the effective search space size. The ligand centre box is useful for ligands to find usual or asymmetric binding modes in the active site or to confine their midpoints into a smaller box to save calculation time. The "centroid of selected residues" option, which specifies the residues that best define the active site was also used, and the inner grid was then centred on the centroid of these selected residues.

### • Receptor-based ligand docking

The final docking algorithm utilised in this study is the Glide SP-algorithm, better known as the standard precision. The nature of docking simulations employed by this algorithm are the same to that of the High Throughput Virtual Screening (HTVS), except that HTVS reduces the number of intermediate conformations throughout the docking funnel, the thoroughness of the final torsional refinement and sampling. During the docking process, the domain-structures were kept rigid (not even the hydroxyl and thiol groups could rotate), and flexibility was induced to all docking ligands. This was achieved through the ligand preparation wizard, which had generated a collection of multiple poses of the ligand database (Zdd). The entire work was done using a Core i7 with 4 cores, 8 processors, and 8GB of RAM.

### RESULTS

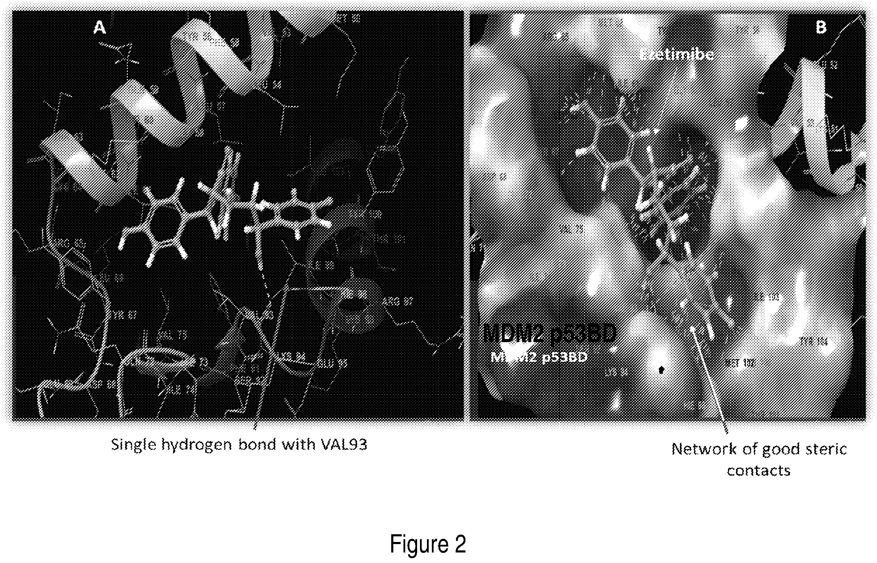

Screening of the Zdd revealed very good binding of ezetimibe to the Mdm2-p53 binding pocket including a hydrogen bond with VAL93. Further molecular docking studies produced the interaction elements shown in **Figure 2** where ezetimibe is docked into the p53-binding domain of Mdm2 i.e. the hydrophobic pocket. In **Figure 2A** a single hydrogen bond with VAL93 and possible hydrophobic interactions are observed. In **Figure 2B** a residue property surface along with a network of good steric contacts of the Mdm2-ezetimibe complex is observed. This surface representation shows the pocket-like nature of the binding site and how ezetimibe accurately mimics the three critical p53 binding residues (PHE19, TRP23, and LEU26).

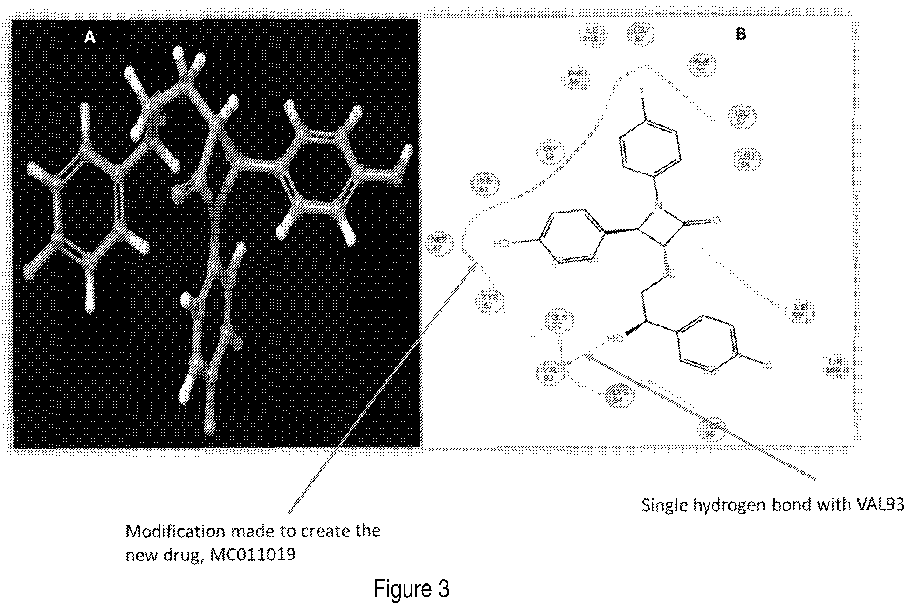

The ligand interaction diagram **(****Figure 3****)** shows that ezetimibe binds tightly to the Mdm2-p53BD hydrophobic pocket. Furthermore, it shows ionic interactions at 3Å axis around the drug **(****Figure 2****).** This finding is likely to have significant impact in cancers with high Mdm2 expression. A fluorine is introduced as shown by the square in **Figure 3** and *in silico* pharmacokinetic studies were conducted. This substitution is critical because it blocks the conversion of the new drug by glucuronidation in the intestines. This modification also improves the druglikeness of the lead compound. One piece of evidence is that the lipophilicity (ClogP) of the drug is increased to 5.04. For ezetimibe lipophilicity did not improve oral bioavailability because after glucuronidation, ezetimibe is excreted through the digestive tract.

In **Figure 4** the structural interaction of MC011019 docked into the MDM2's hydrophobic pocket is depicted, together with the 2D ligand interaction diagram which shows the binding co-ordination at 3Å axis. This binding mode accurately mimics the p53 transactivation domain because the three *para* fluoro-phenyl groups of MC011019 directly bind and occupy the three critical residues (Phe19, Trp23 and Leu26) that facilitate the binding co-ordination of p53. In addition, MC011019 also forms a hydrogen bond with VAL93 similar to that formed by p53 upon binding the MDM2 hydrophobic pocket. Furthermore, this binding mode has a slightly higher docking score of -7.89kJ/mol compared to the -7.76kJ/mol depicted by ezetimibe.

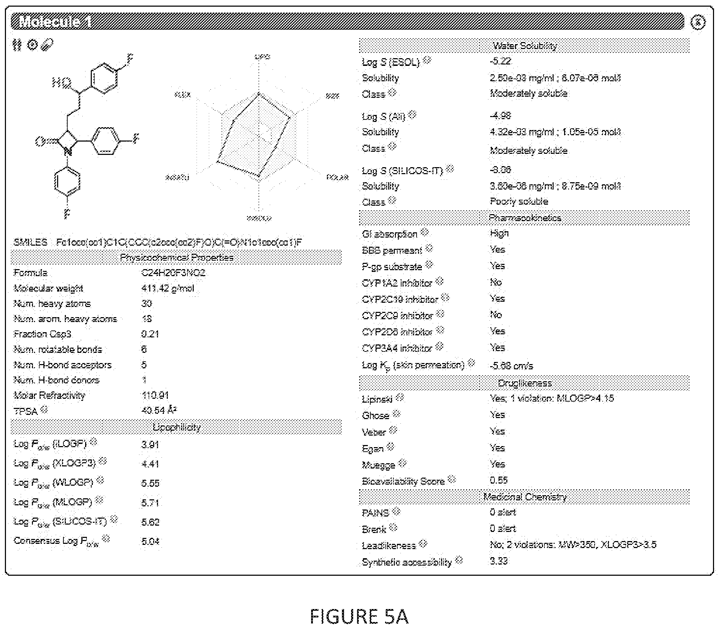

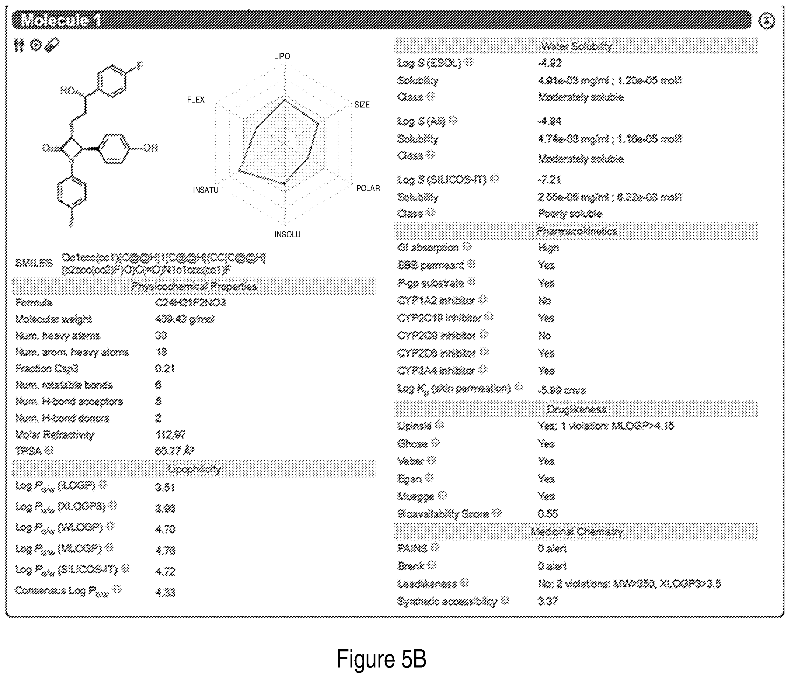

In **Figure 5** the pharmacokinetics (PK) and drug-likeness of MC011019 **(****Figure 5A****)** and ezetimibe **(****Figure 5B****)** are illustrated. The figure was computed using Swiss-ADME.

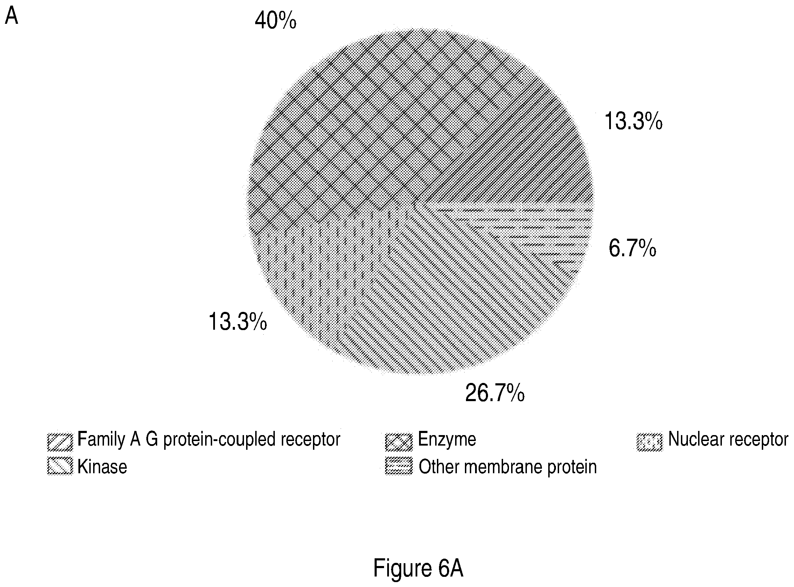

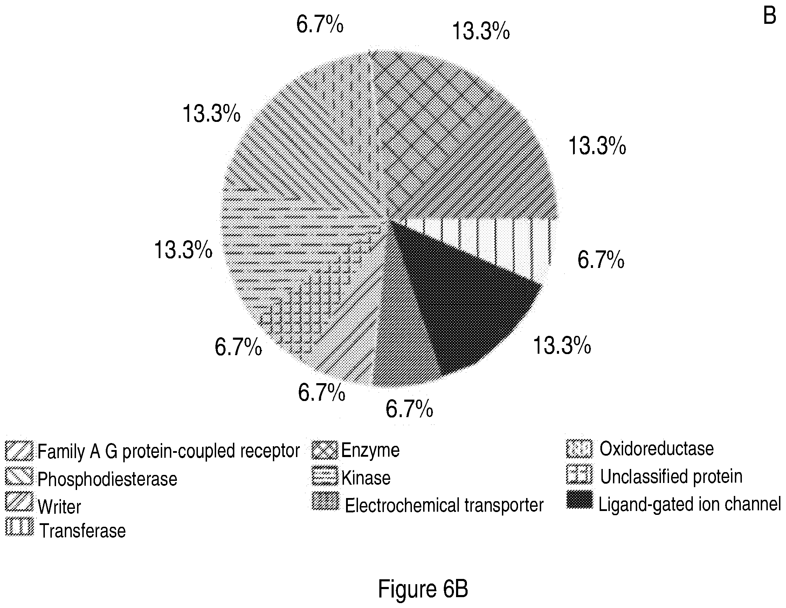

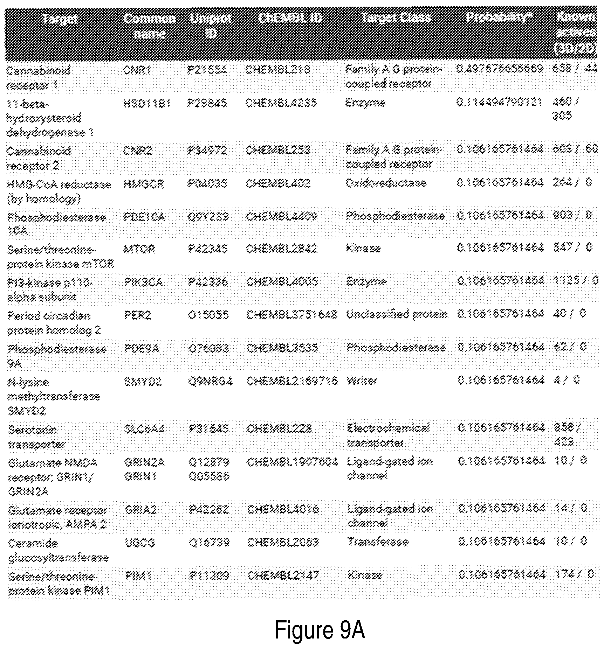

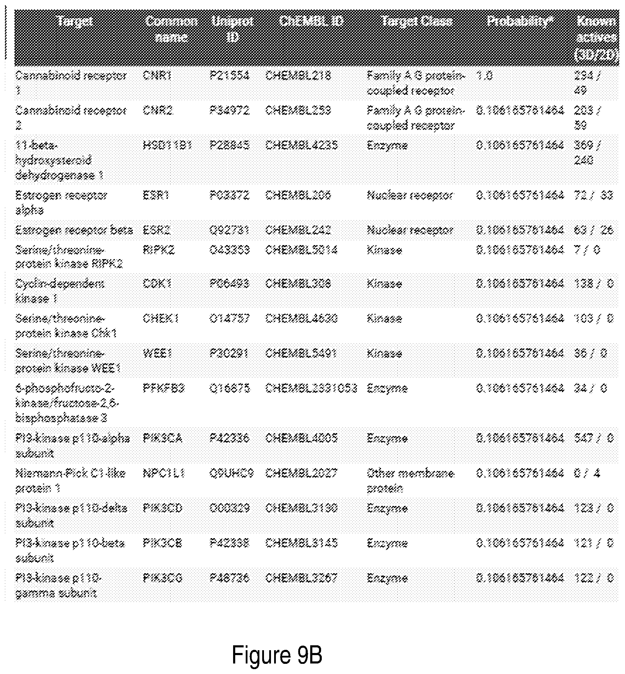

A target prediction experiment shows that MC011019 and ezetimibe have similar biological targets **(****Figure 6****,** **Figure 9A** and **Figure 9B****).** It is noteworthy that MC011019 is not predicted to bind to the ezetimibe cholesterol-related receptor Niemann-Pick C1 Like protein. This means that MC011019 will probably not replicate the current application of ezetimibe. Alternatively, cholesterolaemia is unlikely to be an indication for MC011019 as this depends upon binding to the Niemann-Pick C1 Like protein. Furthermore, the probability of MC011019 to bind cannabidiol receptor 1 has also decreased quite significantly when compared to that of ezetimibe.

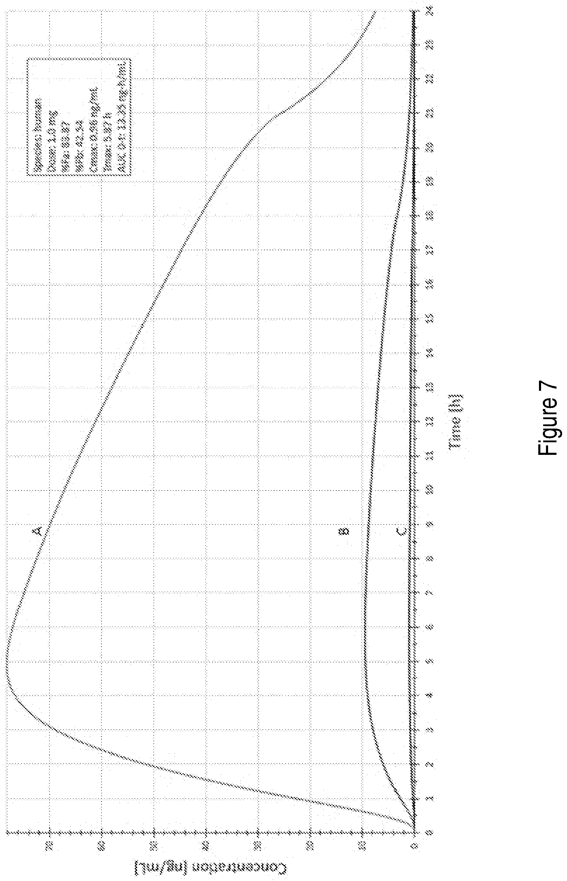

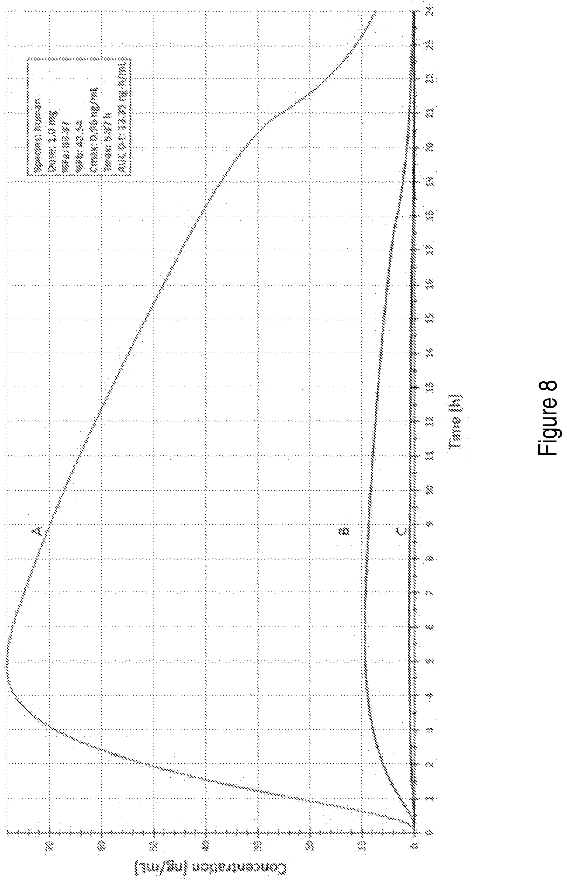

In **Figure 7** **and** **8** the plasma concentration vs time graphs of MC011019 is depicted. Figures 7 and 8 were computed using ADMET PREDICTOR v9.5 from *Simulation Plus, Inc.* These graphs illustrate the pharmacokinetic profile prediction of MC011019 administered at different doses, i.e. 0.01mg or 1mg (C), 0.1mg or 10mg (B) and 1.0mg or 100mg (A) using the rat and human models respectively. This simulation also computes the percentage of fraction absorbed (%Fa) and bioavailable (%Fb) as well as the C-max with its corresponding T-max. It is also worth noting that in both models MC011019 has shown a great absorption and elimination rates, as well as a good %Fb in humans. These observations are also supported by the findings in Figure 6, Figure 9A and Figure 9B which show that MC011019 does not bind the Niemann-Pick C1 Like 1 protein and further possess a very low probability to interact off-target with other cellular proteins.

### DISCUSSION

The *in-silico* studies show that MC011019 binds strongly in the Mdm2-p53 hydrophobic pocket. The increased lipophilicity (LogP_{o/w}) indicates that MC011019 will have a better bioavailability than the parent molecule ezetimibe due to the addition of the fluorine. The replacement of the hydroxyl by the fluorine is necessary to prevent the metabolic conversion of the drug in the small intestine. Another significant observation is that MC011019 probably does not interact with the Niemann-Pick C1 protein which facilitates absorption of cholesterol.

These studies suggest that MC011019 will prevent the binding of Mdm2 to the tumour suppressor protein p53 thereby reactivating p53 for its positive action on cancer cells. It is anticipated that MC011019 will be effective against wild type p53 cancers and in cancers that overexpress Mdm2. In particular, it is anticipated that MC011019 will be effective in targeting colon and colorectal cancers since it is not vulnerable to degradation by intestinal metabolism.

## Claims

1. A compound having the structure of Formula (I): 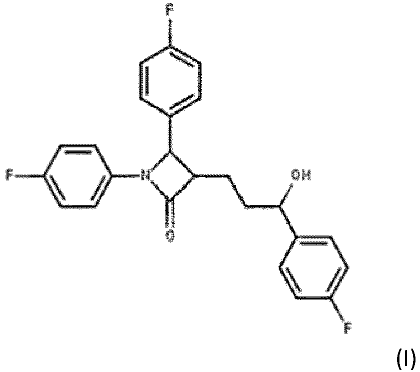 or a pharmaceutically acceptable salt thereof.

2. The compound of claim 1 or a pharmaceutically acceptable salt thereof, wherein the compound binds to a p53 binding pocket of Mdm2 to prevent binding of Mdm2 to p53 and increase p53 levels in a cell.

3. A compound of Formula (I) or a pharmaceutically acceptable salt thereof for use in a method of treating a cancer in which there are elevated levels of Mdm2 or where Mdm2 is overexpressed.

4. The compound or a pharmaceutically acceptable salt thereof for use of claim 3, wherein the cancer is selected from colon cancer, colorectal cancer, sarcoma, glioma, lymphoma, breast cancer, lung cancer, liver cancer, esophagogastric cancer and gynaecological cancer.

5. The compound or a pharmaceutically acceptable salt thereof for use of claim 3 or 4, wherein the compound or a pharmaceutically acceptable salt thereof binds to a p53 binding pocket of Mdm2 to increase levels of p53 in a cell.

## Patentansprüche

1. Verbindung mit dem Aufbau nach Formel (I)
oder ein pharmazeutisch geeignetes Salz davon.

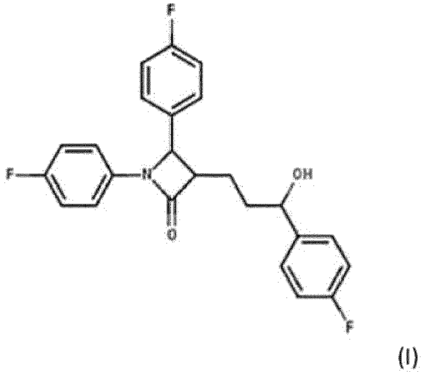

2. Verbindung nach Anspruch 1 oder ein pharmazeutisch geeignetes Salz davon, wobei die Verbindung an eine p53-Bindungsstelle von Mdm2 bindet, um eine Bindung von Mdm2 an p53 zu verhindern und eine Zunahme von p53-Spiegeln in einer Zelle zu erhöhen.

3. Verbindung nach Formel (I) oder ein pharmazeutisch geeignetes Salz davon zur Verwendung in einem Verfahren zur Behandlung von Krebs, wobei erhöhte Pegel von Mdm2 vorhanden sind oder wobei Mdm2 über-exprimiert ist.

4. Verbindung oder pharmazeutisch geeignetes Salz davon zur Verwendung nach Anspruch 3, wobei der Krebs ausgewählt ist aus: Dickdarmkrebs, kolonrektalem Krebs, Sarkom, Gliom, Lymphom, Brustkrebs, Lungenkrebs, Leberkrebs, Speiseröhren-Magenkrebs und Unterleibskrebs.

5. Verbindung oder pharmazeutisch geeignetes Salz davon zur Verwendung nach Anspruch 3 oder 4, wobei die Verbindung oder ein pharmazeutisch geeignetes Salz davon an eine p53-Bindungsstelle von Mdm2 zur Erhöhung von p53-Spiegeln in einer Zelle bindet.

## Revendications

1. Composé ayant une structure de formule (I) : 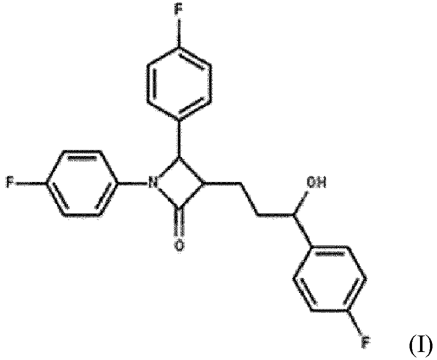 ou un sel pharmaceutiquement acceptable de celui-ci.

2. Composé selon la revendication 1 ou un sel pharmaceutiquement acceptable de celui-ci, dans lequel le composé se lie à une poche de liaison à p53 de Mdm2 pour empêcher la liaison de Mdm2 à p53 et augmenter les niveaux de p53 dans une cellule.

3. Composé selon la formule (I) ou un sel pharmaceutiquement acceptable de celui-ci pour une utilisation dans une méthode de traitement d'un cancer dans lequel il y a des niveaux élevés de Mdm2 ou bien dans lequel Mdm2 est surexprimée.

4. Composé ou un sel pharmaceutiquement acceptable de celui-ci pour une utilisation selon la revendication 3, dans lequel le cancer est choisi parmi un cancer du côlon, un cancer colorectal, un sarcome, un gliome, un lymphome, un cancer du sein, un cancer du poumon, un cancer du foie, un cancer gastro-oesophagique, et un cancer gynécologique.

5. Composé ou un sel pharmaceutiquement acceptable de celui-ci pour une utilisation selon la revendication 3 ou 4, lequel composé ou sel pharmaceutiquement acceptable de celui-ci se lie à une poche de liaison à p53 de Mdm2 pour augmenter les niveaux de p53 dans une cellule.
